(19) Europäisches Patentamt
European Patent Office
Office européen des brevets

(11) EP 3 960 795 A1

(12) EUROPEAN PATENT APPLICATION
published in accordance with Art. 153(4) EPC

(43) Date of publication:
02.03.2022 Bulletin 2022/09

(51) International Patent Classification (IPC):
$C08J\ 3/24^{(2006.01)}$      $A61F\ 13/53^{(2006.01)}$
$C08J\ 3/12^{(2006.01)}$

(21) Application number: 20794330.9

(22) Date of filing: 16.04.2020

(52) Cooperative Patent Classification (CPC):
A61F 13/53; C08J 3/12; C08J 3/24

(86) International application number:
PCT/JP2020/016751

(87) International publication number:
WO 2020/218163 (29.10.2020 Gazette 2020/44)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 23.04.2019 JP 2019082027

(71) Applicant: Sumitomo Seika Chemicals Co., Ltd.
Kako-gun, Hyogo 675-0145 (JP)

(72) Inventors:
• NISHIDA, Moe
Himeji-shi, Hyogo 672-8076 (JP)
• TANIGUCHI, Misaki
Himeji-shi, Hyogo 672-8076 (JP)

(74) Representative: Vossius & Partner
Patentanwälte Rechtsanwälte mbB
Siebertstraße 3
81675 München (DE)

(54) **WATER-ABSORBENT RESIN PARTICLES, ABSORBENT BODY, AND ABSORBENT ARTICLE**

(57) Disclosed is water-absorbent resin particles in which a deterioration rate in terms of 3-minute values of non-pressurization DW measured by the following procedure is 15% or less. The 3-minute value of non-pressurization DW is measured by the following procedure. (1) 15 g of water-absorbent resin particles is disposed in a mixing container having a volume of 1 L and made of stainless steel. (2) Deterioration treatment is performed on the water-absorbent resin particles by pivoting the mixing container in which the water-absorbent resin particles are disposed for 2 hours under conditions of a rotation speed of 45 rpm and a revolution speed of 45 rpm. (3) A deterioration rate in terms of 3-minute values of non-pressurization DW calculated by the following formula is obtained, in which I is a 3-minute value of non-pressurization DW of the water-absorbent resin particles before the deterioration treatment and II is a 3-minute value of non-pressurization DW of the water-absorbent resin particles after the deterioration treatment.

Formula: $[(I - II)/I] \times 100$

Fig.2

**Description**

**Technical Field**

The present invention relates to water-absorbent resin particles, an absorber, and an absorbent article.

**Background Art**

In the related art, an absorber containing water-absorbent resin particles has been used in an absorbent article for absorbing a liquid (for example, urine) having water as a main component. For example, Patent Literature 1 below discloses water-absorbent resin particles having a particle diameter that is suitably used for absorbent articles such as diapers. In addition, Patent Literature 2 discloses a method of using a hydrogel-absorbent polymer having specific saline flow inducibility, performance under pressure, and the like as an effective absorbent member for storing a body fluid such as urine.

**Citation List**

**Patent Literature**

    Patent Literature 1: Japanese Unexamined Patent Publication No. H06-345819

    Patent Literature 2: Published Japanese Translation No. H09-510889 of the PCT International Publication

**Summary of Invention**

**Technical Problem**

It is desirable that occurrence of liquid leakage from an absorbent article during use is suppressed as much as possible.
An object of an aspect of the present invention is to provide water-absorbent resin particles capable of obtaining an absorbent article from which liquid leakage during use is suppressed. An object of another aspect of the present invention is to provide an absorber and an absorbent article using the water-absorbent resin particles.

**Solution to Problem**

An aspect of the present invention relates to water-absorbent resin particles in which a deterioration rate in terms of 3-minute values of non-pressurization DW is 15% or less. The deterioration rate in terms of 3-minute values of non-pressurization DW is measured by the following procedure.

    (1) 15 g of water-absorbent resin particles is disposed in a mixing container having a volume of 1 L and made of stainless steel.
    (2) Deterioration treatment is performed on the water-absorbent resin particles by pivoting the mixing container in which the water-absorbent resin particles are disposed for 2 hours under conditions of a rotation speed of 45 rpm and a revolution speed of 45 rpm.
    (3) A deterioration rate in terms of 3-minute values of non-pressurization DW is calculated by the following formula is obtained, when I is a 3-minute value of non-pressurization DW of the water-absorbent resin particles before the deterioration treatment and II is a 3-minute value of non-pressurization DW of the water-absorbent resin particles after the deterioration treatment.

$$[(I - II)/I] \times 100$$

According to the above-mentioned water-absorbent resin particles, it is possible to obtain an absorbent article from which liquid leakage during use is suppressed.
Another aspect of the present invention provides an absorber containing the above-mentioned water-absorbent resin particles. Still another aspect of the present invention provides an absorbent article including the above-mentioned absorber.

**Advantageous Effects of Invention**

According to an aspect of the present invention, it is possible to provide water-absorbent resin particles capable of obtaining an absorbent article from which liquid leakage during use is suppressed. According to another aspect of the present invention, it is possible to provide an absorber and an absorbent article using the water-absorbent resin particles. According to still another aspect of the present invention, it is possible to provide use of resin particles, an absorber, and an absorbent article to absorption of a liquid.

**Brief Description of Drawings**

Fig. 1 is a cross-sectional view showing an embodiment of an absorbent article.
Fig. 2 is a plan view showing an outline of a stirring blade used in Examples.
Fig. 3 is a schematic view showing an example of a method for measuring non-pressurization DW.
Fig. 4 is a schematic view showing an example of a method for measuring a deterioration rate.

**Description of Embodiments**

Hereinafter, some embodiments of the present invention will be described in detail. However, the present invention is not limited to the following embodiments.

In the present specification, "acrylic" and "methacrylic" are collectively referred to as "(meth)acrylic". "Acrylate" and "methacrylate" are also referred to as "(meth)acrylate". "(Poly)" means both of a case where there is a prefix of "poly" and a case where there is no prefix thereof. In a numerical value range described in a stepwise manner in the present specification, an upper limit value or a lower limit value of a numerical value range in a certain step can be arbitrarily combined with an upper limit value or a lower limit value of a numerical value range in another step. In a numerical value range described in the present specification, an upper limit value or a lower limit value of the numerical value range may be replaced with a value shown in Examples. "A or B" means that any one of A and B may be contained, and both thereof may be contained. "Water-soluble" means that a solubility of 5% by mass or more is exhibited in water at 25°C. For materials exemplified in the present specification, one kind may be used alone, or two or more kinds may be used in combination. In a case where there are a plurality of substances corresponding to each of components in a composition, a content of each of the components in the composition means the total amount of the plurality of substances present in the composition unless otherwise specified.

A deterioration rate of water-absorbent resin particles of an embodiment in terms of 3-minute values of non-pressurization DW is 15% or less, which is measured by the following procedure.

(1) 15 g of water-absorbent resin particles is disposed in a mixing container having a volume of 1 L and made of stainless steel.
(2) Deterioration treatment is performed on the water-absorbent resin particles by pivoting the mixing container in which the water-absorbent resin particles are disposed for 2 hours under conditions of a rotation speed of 45 rpm and a revolution speed of 45 rpm.
(3) A deterioration rate in terms of 3-minute values of non-pressurization DW calculated by the following formula is obtained, when I is a 3-minute value of non-pressurization DW of the water-absorbent resin particles before the deterioration treatment and II is a 3-minute value of non-pressurization DW of the water-absorbent resin particles after the deterioration treatment.

$$[(\mathrm{I} - \mathrm{II})/\mathrm{I}] \times 100$$

When the deterioration rate in terms of 3-minute values of non-pressurization DW is small, liquid leakage during use of an absorbent article using the water-absorbent resin particles tends to be more effectively suppressed.

The deterioration rate may be 14% or less, 13% or less, 12% or less, 11% or less, 10% or less, 9% or less, 8% or less, 7% or less, 6% or less, 5% or less, 4% or less, 3% or less, 2% or less, or 1% or less. The deterioration rate may be 0%. That is, in the water-absorbent resin particles of an embodiment, the 3-minute value of non-pressurization DW before and after the deterioration treatment may not be changed.

For example, the water-absorbent resin particles showing the deterioration rate of 15% or less can be obtained by adjusting the crosslinking density in the inside and/or surface layer of the water-absorbent resin particles (for example, uniformly performing crosslinking).

The deterioration rate in terms of 3-minute values of non-pressurization DW can be specifically measured by a method described in Examples to be described later. In measurement of the deterioration rate in terms of 3-minute values of non-pressurization DW, as a mixing container, it is possible to use a container having the inlet inner diameter of 4.1 cm, the body inner diameter of 10.0 cm, and the height of 20.6 cm, for example. The deterioration treatment is performed using a mixer in which a rotation speed and a revolution speed can be adjusted in the above-mentioned range. Examples of the mixer include, but are not limited to, a cross rotary mixer (CM-3 type) manufactured by Meiwa Kogyo Co., Ltd. or Tsukasa Industry Co., Ltd.

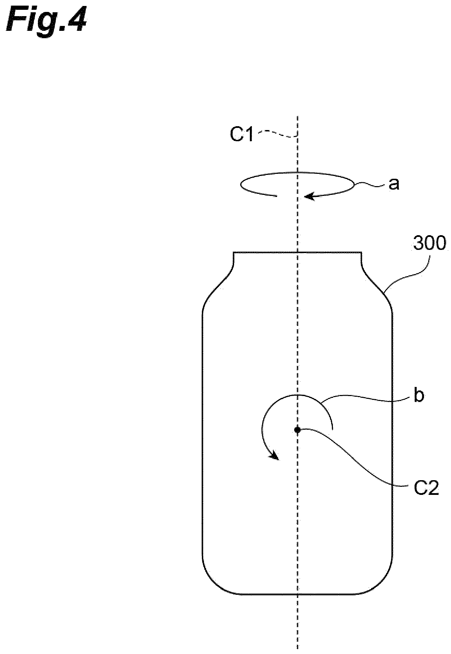

Fig. 4 is a schematic view for showing directions of rotation and revolution of the mixing container. A mixing container 300 is pivoted in a state where the bottom surface and the upper surface of the mixing container 300 are rotatably and revolvably fixed. As shown in Fig. 4, the mixing container 300 revolves in the direction of b around a revolution axis C2 that is located at the center in a height direction of the mixing container 300 and is orthogonal to a rotation axis C1 that is a central axis in a height direction of the mixing container 300 while rotating in the direction of a (clockwise direction when viewed from the upper surface side of the mixing container) around the rotation axis C1.

The 3-minute value of non-pressurization DW (3-minute value of non-pressurization DW before the deterioration treatment) of the water-absorbent resin particles according to the present embodiment may be, for example, 5 mL/g or more or 10 mL/g or more, and may be 60 mL/g or less, 55 mL/g or less, 50 mL/g or less, 40 mL/g or less, or 30 mL/g or less.

The 10-minute value of non-pressurization DW (10-minute value of non-pressurization DW before the deterioration treatment) of the water-absorbent resin particles according to the present embodiment may be, for example, 25 mL/g or more or 35 mL/g or more, and may be 80 mL/g or less or 70 mL/g or less.

The 3-minute value and 10-minute value of non-pressurization DW are measured by a method described in Examples to be described later.

The water-absorbent resin particles of the present embodiment may be any water-absorbent resin particles as long as the water-absorbent resin particles can retain water, and the liquid to be absorbed can contain water. The water-absorbent resin particles of the present embodiment are better in absorbency of a body fluid such as urine, sweat, blood (for example, menstrual blood). The water-absorbent resin particles of the present embodiment can be used as a constituent component of the absorber of the present embodiment.

The physiological saline retention amount of the water-absorbent resin particles of the present embodiment may be 20 g/g or more, 25 g/g or more, 30 g/g or more, 35 g/g or more, more than 40 g/g, or 41 g/g or more. The physiological saline retention amount of the water-absorbent resin particles of the present embodiment may be 80 g/g or less, 75 g/g or less, 70 g/g or less, 65 g/g or less, 60 g/g or less, or 55 g/g or less. For example, the physiological saline retention amount of the water-absorbent resin particles of the present embodiment may be 20 to 80 g/g, 30 to 70 g/g, 35 to 60 g/g, or 40 to 55 g/g. The water retention amount may be a water retention amount at room temperature (25°C $\pm$ 2°C). The water retention amount can be measured by a method described in Examples to be described later.

Examples of the shape of the water-absorbent resin particles of the present embodiment include a substantially spherical shape, a crushed shape, and a granular shape. Furthermore, the water-absorbent resin particles of the present embodiment may be in the form (secondary particles) in which fine particles (primary particles) are aggregated, in addition to the form in which each is composed of a single particle. The median particle diameter of the water-absorbent resin particles (water-absorbent resin particles before absorbing water) of the present embodiment is preferably within the following range. The median particle diameter may be 100 $\mu$m or more, 140 $\mu$m or more, 200 $\mu$m or more, 250 $\mu$m or more, 300 $\mu$m or more, 330 $\mu$m or more, or 350 $\mu$m or more. The median particle diameter may be 600 $\mu$m or less, 550 $\mu$m or less, 500 $\mu$m or less, 450 $\mu$m or less, 400 $\mu$m or less, or 380 $\mu$m or less. The median particle diameter may be 100 to 600 $\mu$m, for example. The water-absorbent resin particles of the present embodiment may have a desired particle size distribution at the time of being obtained by a production method to be described later, but the particle size distribution may be adjusted by performing an operation such as particle size adjustment using classification with a sieve.

The water-absorbent resin particles of the present embodiment can contain a crosslinked polymer (a crosslinked polymer having a structural unit derived from an ethylenically unsaturated monomer) obtained by polymerizing a monomer containing an ethylenically unsaturated monomer, as polymer particles, for example. That is, the water-absorbent resin particles of the present embodiment can have a polymer having a structural unit derived from an ethylenically unsaturated monomer, and can contain polymer particles including a crosslinked polymer having a structural unit derived from an ethylenically unsaturated monomer. As the ethylenically unsaturated monomer, a water-soluble ethylenically unsaturated monomer can be used. Examples of the polymerization method include a reverse phase suspension polymerization method, an aqueous solution polymerization method, a bulk polymerization method, and a precipitation polymerization method. Among these, the reverse phase suspension polymerization method or the aqueous solution polymerization method is preferable from the viewpoint of ensuring good water-absorbent characteristics (such as a water retention amount) of the obtained water-absorbent resin particles and facilitating control of the polymerization reaction. In the following, as a method for polymerizing an ethylenically unsaturated monomer, a reverse phase suspension polymerization method will be described as an example.

The ethylenically unsaturated monomer is preferably water-soluble, and examples thereof include (meth)acrylic acid and a salt thereof, 2-(meth)acrylamide-2-methylpropanesulfonic acid and a salt thereof, (meth)acrylamide, N,N-dimethyl (meth)acrylamide, 2-hydroxyethyl (meth)acrylate, N-methylol (meth)acrylamide, polyethylene glycol mono(meth)acrylate, N,N-diethylaminoethyl (meth)acrylate, N,N-diethylaminopropyl (meth)acrylate, and diethylaminopropyl (meth)acrylamide. In a case where the ethylenically unsaturated monomer has an amino group, the amino group may be quaternized. The ethylenically unsaturated monomer may be used alone, or may be used in combination of two or more kinds thereof. The functional group, such as a carboxyl group and an amino group, of the above-mentioned monomer can function as a functional group capable of crosslinking in a surface crosslinking step to be described later.

Among these, from the viewpoint of industrial availability, the ethylenically unsaturated monomer preferably contains at least one compound selected from the group consisting of (meth)acrylic acid and a salt thereof, acrylamide, methacrylamide, and N,N-dimethyl acrylamide, and more preferably contains at least one compound selected from the group consisting of (meth)acrylic acid and a salt thereof, and acrylamide. From the viewpoint of further enhancing water-absorbent characteristics (such as a water retention amount), the ethylenically unsaturated monomer further more preferably contains at least one compound selected from the group consisting of (meth)acrylic acid and a salt thereof. That is, the water-absorbent resin particles preferably contain a polymer having at least one structural unit selected from the group consisting of a structural unit derived from (meth)acrylic acid and a structural unit derived from a salt of (meth)acrylic acid.

As the monomer for obtaining the water-absorbent resin particles, a monomer other than the above-mentioned ethylenically unsaturated monomer may be used. Such a monomer can be used by being mixed with an aqueous solution containing the above-mentioned ethylenically unsaturated monomer, for example. The use amount of the ethylenically unsaturated monomer may be 70 to 100 mol%, and may be 80 to 100 mol%, 90 to 100 mol%, 95 to 100 mol%, or 100 mol% with respect to the total amount of the monomer (the total amount of the monomer for obtaining the water-absorbent resin particles. For example, the total amount of the monomers that provide a structural unit of the crosslinked polymer. The same applies hereinafter). Among these, the ratio of (meth)acrylic acid and a salt thereof may be 70 to 100 mol%, and may be 80 to 100 mol%, 90 to 100 mol%, 95 to 100 mol%, or 100 mol% with respect to the total amount of the monomers. "Ratio of (meth)acrylic acid and a salt thereof' means the ratio of the total amount of (meth)acrylic acid and a salt thereof.

According to the present embodiment, as an example of the water-absorbent resin particles, it is possible to provide the water-absorbent resin particle containing a crosslinked polymer having a structural unit derived from an ethylenically unsaturated monomer, in which the ethylenically unsaturated monomer contains at least one compound selected from the group consisting of (meth)acrylic acid and a salt thereof, and the ratio of (meth)acrylic acid and a salt thereof is 70 to 100 mol% with respect to the total amount of the monomer for obtaining the water-absorbent resin particles.

The ethylenically unsaturated monomer is usually preferably used as an aqueous solution. The concentration of the ethylenically unsaturated monomer in the aqueous solution containing the ethylenically unsaturated monomer (hereinafter, simply referred to as "monomer aqueous solution") is preferably 20% by mass or more and a saturated concentration or less, more preferably 25 to 70% by mass, and further more preferably 30 to 55% by mass. Examples of the water used in the aqueous solution include tap water, distilled water, and ion-exchanged water.

In a case where the ethylenically unsaturated monomer has an acid group, the monomer aqueous solution may be used by neutralizing the acid group with an alkaline neutralizing agent. The degree of neutralization of the ethylenically unsaturated monomer by the alkaline neutralizing agent is preferably 10 to 100 mol%, more preferably 50 to 90 mol%, and further more preferably 60 to 80 mol% of the acid group in the ethylenically unsaturated monomer, from the viewpoint of increasing an osmotic pressure of the obtained water-absorbent resin particles, and further enhancing water-absorbent characteristics (such as a water retention amount). Examples of the alkaline neutralizing agent include alkali metal salts such as sodium hydroxide, sodium carbonate, sodium hydrogen carbonate, potassium hydroxide, and potassium carbonate; and ammonia. The alkaline neutralizing agent may be used alone, or may be used in combination of two or more kinds thereof. The alkaline neutralizing agent may be used in the form of an aqueous solution to simplify the neutralization operation. Neutralization of the acid group of the ethylenically unsaturated monomer can be performed by adding an aqueous solution of sodium hydroxide, potassium hydroxide, or the like dropwise in the above-mentioned monomer aqueous solution and mixing therewith.

In a reverse phase suspension polymerization method, a monomer aqueous solution is dispersed in a hydrocarbon dispersion medium in the presence of a surfactant, and polymerization of the ethylenically unsaturated monomer can be performed using a radical polymerization initiator or the like. As the radical polymerization initiator, a water-soluble radical polymerization initiator can be used.

Examples of the surfactant include a nonionic surfactant and an anionic surfactant. Examples of the nonionic surfactant include sorbitan fatty acid esters, polyglycerin fatty acid esters, sucrose fatty acid esters, polyoxyethylene sorbitan fatty acid esters, polyoxyethylene glycerin fatty acid esters, sorbitol fatty acid esters, polyoxyethylene sorbitol fatty acid esters, polyoxyethylene alkyl ethers, polyoxyethylene alkylphenyl ethers, polyoxyethylene castor oil, polyoxyethylene hydrogenated castor oil, alkylallyl formaldehyde condensed polyoxyethylene ethers, polyoxyethylene polyoxypropylene block copolymers, polyoxyethylene polyoxypropyl alkyl ethers, and polyethylene glycol fatty acid esters. Examples of the anionic surfactant include fatty acid salts, alkylbenzene sulfonate, alkylmethyl taurate, polyoxyethylene alkylphenyl ether sulfuric acid ester salts, polyoxyethylene alkyl ether sulfonic acid salts, phosphoric acid esters of polyoxyethylene alkyl ethers, and phosphoric acid esters of polyoxyethylene alkyl allyl ethers. The surfactant may be used alone, or may be used in combination of two or more kinds thereof.

From the viewpoint of a good state of the W/O type reverse phase suspension, easily obtaining water-absorbent resin particles having a suitable particle diameter, and industrial availability, the surfactant preferably contains at least one compound selected from the group consisting of sorbitan fatty acid esters, polyglycerin fatty acid esters, and sucrose fatty acid esters. From the viewpoint of easily obtaining an appropriate particle size distribution of the water-absorbent resin particles, and from the viewpoint of easily improving water-absorbent characteristics (such as a water retention amount) of the water-absorbent resin particles and performances of the absorbent article using the same, the surfactant preferably contains sucrose fatty acid ester, and more preferably contains sucrose stearic acid ester.

The use amount of the surfactant is preferably 0.05 to 10 parts by mass, more preferably 0.08 to 5 parts by mass, and further more preferably 0.1 to 3 parts by mass with respect to 100 parts by mass of the monomer aqueous solution, from the viewpoint of obtaining a sufficient effect on the use amount and economic efficiency.

In the reverse phase suspension polymerization, a polymeric dispersant may be used in combination with the above-mentioned surfactant. Examples of the polymeric dispersant include maleic anhydride-modified polyethylene, maleic anhydride-modified polypropylene, a maleic anhydride-modified ethylene-propylene copolymer, a maleic anhydride-modified EPDM (ethylene propylene diene terpolymer), maleic anhydride-modified polybutadiene, a maleic anhydride-ethylene copolymer, a maleic anhydride-propylene copolymer, a maleic anhydride-ethylene-propylene copolymer, a maleic anhydride-butadiene copolymer, polyethylene, polypropylene, an ethylene-propylene copolymer, oxidized polyethylene, oxidized polypropylene, an oxidized ethylene-propylene copolymer, an ethylene-acrylic acid copolymer, ethyl cellulose, and ethyl hydroxyethyl cellulose. The polymeric dispersant may be used alone or may be used in combination of two or more kinds thereof. From the viewpoint of better dispersion stability of the monomer, the polymeric dispersant is preferably at least one selected from the group consisting of maleic anhydride-modified polyethylene, maleic anhydride-modified polypropylene, a maleic anhydride-modified ethylene-propylene copolymer, a maleic anhydride-ethylene copolymer, a maleic anhydride-propylene copolymer, a maleic anhydride-ethylene-propylene copolymer, polyethylene, polypropylene, an ethylene-propylene copolymer, oxidized polyethylene, oxidized polypropylene, and an oxidized ethylene-propylene copolymer.

The use amount of the polymeric dispersant is preferably 0.05 to 10 parts by mass, more preferably 0.08 to 5 parts by mass, and further more preferably 0.1 to 3 parts by mass with respect to 100 parts by mass of the monomer aqueous solution, from the viewpoint of obtaining a sufficient effect on the use amount and economic efficiency.

The hydrocarbon dispersion medium may contain at least one compound selected from the group consisting of chain aliphatic hydrocarbons having 6 to 8 carbon atoms and alicyclic hydrocarbons having 6 to 8 carbon atoms. Examples of the hydrocarbon dispersion medium include chain aliphatic hydrocarbons such as n-hexane, n-heptane, 2-methylhexane, 3-methylhexane, 2,3-dimethylpentane, 3-ethylpentane, and n-octane; alicyclic hydrocarbons such as cyclohexane, methylcyclohexane, cyclopentane, methylcyclopentane, trans-1,2-dimethylcyclopentane, cis-1,3-dimethylcyclopentane, and trans-1,3-dimethylcyclopentane; and aromatic hydrocarbons such as benzene, toluene, and xylene. The hydrocarbon dispersion medium may be used alone, or may be used in combination of two or more kinds thereof.

The hydrocarbon dispersion medium may contain at least one selected from the group consisting of n-heptane and cyclohexane from the viewpoint of industrial availability and stable quality. In addition, from the same viewpoint, as the mixture of the above-mentioned hydrocarbon dispersion medium, for example, a commercially available Exxsol Heptane (manufactured by ExxonMobil: containing 75% to 85% of n-heptane and isomeric hydrocarbons) may be used.

The use amount of the hydrocarbon dispersion medium is preferably 30 to 1000 parts by mass, more preferably 40 to 500 parts by mass, and further more preferably 50 to 400 parts by mass with respect to 100 parts by mass of the monomer aqueous solution, from the viewpoint of appropriately removing the heat of polymerization and easily controlling the polymerization temperature. In a case where the use amount of the hydrocarbon dispersion medium is 30 parts by mass or more, the polymerization temperature tends to be easily controlled. In a case where the use amount of the hydrocarbon dispersion medium is 1000 parts by mass or less, the productivity of polymerization tends to be improved, which is economical.

The radical polymerization initiator is preferably water-soluble, and examples thereof include persulfates such as potassium persulfate, ammonium persulfate, and sodium persulfate; peroxides such as methyl ethyl ketone peroxide, methyl isobutyl ketone peroxide, di-t-butyl peroxide, t-butyl cumyl peroxide, t-butyl peroxyacetate, t-butyl peroxyisobutyrate, t-butyl peroxypivalate, and hydrogen peroxide; and azo compounds such as 2,2'-azobis(2-amidinopropane) dihydrochloride, 2,2'-azobis[2-(N-phenylamidino)propane] dihydrochloride, 2,2'-azobis[2-(N-allylamidino)propane] dihydrochloride, 2,2'-azobis[2-(2-imidazolin-2-yl)propane] dihydrochloride, 2,2'-azobis{2-[1-(2-hydroxyethyl)-2-imidazolin-2-yl]propane} dihydrochloride,

2,2'-azobis{2-methyl-N-[1,1-bis(hydroxymethyl)-2-hydroxyethyl]propi onamide}, 2,2'-azobis[2-methyl-N-(2-hydroxyethyl)-propionamide], and 4,4'-azobis(4-cyanovaleric acid). The radical polymerization initiator may be used alone, or may be used in combination of two or more kinds thereof. The radical polymerization initiator is preferably at least one selected from the group consisting of potassium persulfate, ammonium persulfate, sodium persulfate, 2,2'-azobis(2-amidinopropane) dihydrochloride, 2,2'-azobis[2-(2-imidazolin-2-yl)propane] dihydrochloride, and 2,2'-azobis{2-[1-(2-hydroxyethyl)-2-imidazolin-2-yl]propane} dihydrochloride; and more preferably potassium persulfate, ammonium persulfate, or sodium persulfate.

The use amount of the radical polymerization initiator may be 0.05 to 10 mmol with respect to 1 mol of the ethylenically unsaturated monomer. In a case where the use amount of the radical polymerization initiator is 0.05 mmol or more, the polymerization reaction does not require a long time and is efficient. In a case where the use amount of the radical polymerization initiator is 10 mmol or less, the occurrence of a rapid polymerization reaction is easily suppressed.

The above-mentioned radical polymerization initiator can also be used as a redox polymerization initiator in combination with a reducing agent such as sodium sulfite, sodium hydrogen sulfite, ferrous sulfate, and L-ascorbic acid.

At the time of the polymerization reaction, the monomer aqueous solution used for the polymerization may contain a chain transfer agent. Examples of the chain transfer agent include hypophosphites, thiols, thiolic acids, secondary alcohols, and amines.

The monomer aqueous solution used for the polymerization may contain a thickener in order to control the particle diameter of the water-absorbent resin particles. Examples of the thickener include hydroxyethyl cellulose, hydroxypropyl cellulose, methyl cellulose, carboxymethyl cellulose, polyethylene glycol, polyacrylamide, polyethyleneimine, dextrin, sodium alginate, polyvinyl alcohol, polyvinylpyrrolidone, and polyethylene oxide. In a case where the stirring speed at the time of polymerization is the same, the higher the viscosity of the monomer aqueous solution, the larger the median particle diameter of the obtained particles tends to be.

Internal crosslinking by self-crosslinking may occur during polymerization, but crosslinking may be performed by using an internal crosslinking agent. In a case where an internal crosslinking agent is used, water-absorbent characteristics (such as a water retention amount) of the water-absorbent resin particles are easily controlled. The internal crosslinking agent is usually added to a reaction solution during the polymerization reaction. Examples of the internal crosslinking agent include di or tri (meth)acrylic acid esters of polyols such as ethylene glycol, propylene glycol, trimethylolpropane, glycerin, polyoxyethylene glycol, polyoxypropylene glycol, and polyglycerin; unsaturated polyesters obtained by reacting the above-mentioned polyols with unsaturated acids (such as maleic acid and fumaric acid); bis(meth)acrylamides such as N,N'-methylenebis(meth)acrylamide; di or tri (meth)acrylic acid esters obtained by reacting a polyepoxide with (meth)acrylic acid; carbamyl di(meth)acrylate esters obtained by reacting a polyisocyanate (such as tolylene diisocyanate and hexamethylene diisocyanate) with hydroxyethyl (meth)acrylate; compounds having two or more polymerizable unsaturated groups, such as allylated starch, allylated cellulose, diallyl phthalate, N,N',N''-triallyl isocyanurate, and divinylbenzene; polyglycidyl compounds such as (poly)ethylene glycol diglycidyl ether, (poly)propylene glycol diglycidyl ether, (poly)glycerin diglycidyl ether, (poly)glycerin triglycidyl ether, (poly)propylene glycol polyglycidyl ether, and polyglycerol polyglycidyl ether; haloepoxy compounds such as epichlorohydrin, epibromohydrin, and $\alpha$-methyl epichlorohydrin; and compounds having two or more reactive functional groups, such as isocyanate compounds (such as 2,4-tolylene diisocyanate and hexamethylene diisocyanate). The internal crosslinking agent may be used alone, or may be used in combination of two or more kinds thereof. The internal crosslinking agent is preferably a polyglycidyl compound, is more preferably a diglycidyl ether compound, and is further more preferably at least one selected from the group consisting of (poly)ethylene glycol diglycidyl ether, (poly)propylene glycol diglycidyl ether, and (poly)glycerin diglycidyl ether.

The use amount of the internal crosslinking agent is preferably 30 mmol or less, more preferably 0.01 to 10 mmol, further more preferably 0.012 to 5 mmol, particularly preferably 0.015 to 1 mmol, extremely preferably 0.02 to 0.1 mmol, and extraordinarily preferably 0.025 to 0.06 mmol, per 1 mol of the ethylenically unsaturated monomer, from the viewpoint of suppressing water-soluble property by appropriately crosslinking the obtained polymer to easily obtain the sufficient water absorption amount.

It is possible to perform heating while stirring in a state of mixing an ethylenically unsaturated monomer, a radical polymerization initiator, a surfactant, a polymeric dispersant, a hydrocarbon dispersion medium, or the like (if necessary, additionally an internal crosslinking agent), and to perform reverse phase suspension polymerization in a water-in-oil system.

When performing the reverse phase suspension polymerization, a monomer aqueous solution containing an ethylenically unsaturated monomer is dispersed in a hydrocarbon dispersion medium in the presence of a surfactant (if necessary, additionally a polymeric dispersant). At this time, before the start of the polymerization reaction, the timing of adding the surfactant, the polymeric dispersant, or the like may be either before or after the addition of the monomer aqueous solution.

Among these, from the viewpoint of easily reducing the amount of the hydrocarbon dispersion medium remaining in the obtained water-absorbent resin, it is preferable to perform polymerization after dispersing the monomer aqueous solution in the hydrocarbon dispersion medium in which the polymeric dispersant is dispersed and then further dispersing the surfactant.

Reverse phase suspension polymerization can be performed in one stage, or in multiple stages of two or more stages. Reverse phase suspension polymerization is preferably performed in two to three stages from the viewpoint of increasing productivity.

In a case where reverse phase suspension polymerization is performed in multiple stages of two or more stages, a first stage reverse phase suspension polymerization is performed, an ethylenically unsaturated monomer is added to the reaction mixture obtained in the first polymerization reaction and mixed therewith, and second and subsequent stages of reverse phase suspension polymerization may be performed in the same method as the first stage. In the reverse phase suspension polymerization in each stage of the second and subsequent stages, in addition to the ethylenically unsaturated monomer, the above-mentioned radical polymerization initiator and/or internal crosslinking agent is preferably added in a range of a molar ratio of each component with respect to the above-mentioned ethylenically unsaturated monomer, based on an amount of the ethylenically unsaturated monomer added at the time of the second and subsequent stages of reverse phase suspension polymerization, to perform reverse phase suspension polymerization. In the reverse phase suspension polymerization in each stage of second and subsequent stages, an internal crosslinking agent may be used if necessary. In a case of using the internal crosslinking agent, the internal crosslinking agent is preferably added within a range of the molar ratio of each component with respect to the above-mentioned ethylenically unsaturated monomer based on the amount of the ethylenically unsaturated monomer provided in each stage, to perform reverse phase suspension polymerization.

The temperature of the polymerization reaction varies depending on the used radical polymerization initiator, and the temperature is preferably 20°C to 150°C, and more preferably 40°C to 120°C, from the viewpoint of rapidly proceeding the polymerization and shortening the polymerization time to enhance economic efficiency, and easily removing polymerization heat and smoothly performing reaction. The reaction time is usually 0.5 to 4 hours. The completion of the polymerization reaction can be confirmed by stopping the temperature rise in the reaction system. Thus, the polymer of the ethylenically unsaturated monomer is usually obtained in a state of a hydrogel.

After the polymerization, a post-polymerization crosslinking agent may be added to the obtained hydrogel-like polymer and heated to perform crosslinking. By performing post-polymerization crosslinking, a degree of crosslinking of the hydrogel-like polymer can be increased, and water-absorbent characteristics (such as a water retention amount) can be further improved.

Examples of the post-polymerization crosslinking agent include polyols such as ethylene glycol, propylene glycol, 1,4-butanediol, trimethylolpropane, glycerin, polyoxyethylene glycol, polyoxypropylene glycol, and polyglycerin; compounds having two or more epoxy groups, such as (poly)ethylene glycol diglycidyl ether, (poly)propylene glycol diglycidyl ether, and (poly)glycerin diglycidyl ether; haloepoxy compounds such as epichlorohydrin, epibromohydrin, and $\alpha$-methyl epichlorohydrin; compounds having two or more isocyanate groups such as 2,4-tolylene diisocyanate and hexamethylene diisocyanate; oxazoline compounds such as 1,2-ethylenebisoxazoline; carbonate compounds such as ethylene carbonate; and hydroxyalkylamide compounds such as bis[N,N-di($\beta$-hydroxyethyl)]adipamide. Among these, polyglycidyl compounds such as (poly)ethylene glycol diglycidyl ether, (poly)glycerin diglycidyl ether, (poly)glycerin triglycidyl ether, (poly)propylene glycol polyglycidyl ether, and polyglycerol polyglycidyl ether are preferable. The crosslinking agent may be used alone, or may be used in combination of two or more kinds thereof.

The amount of the post-polymerization crosslinking agent is preferably 30 mmol or less, more preferably 10 mmol or less, further more preferably 0.01 to 5 mmol, particularly preferably 0.012 to 1 mmol, extremely preferably 0.015 to 0.1 mmol, and extraordinarily preferably 0.02 to 0.05 mmol per 1 mol of the ethylenically unsaturated monomer, from the viewpoint of easily obtaining suitable water-absorbent characteristics (such as a water retention amount).

The timing of adding the post-polymerization crosslinking agent may be after the polymerization of the ethylenically unsaturated monomer used for the polymerization, and in the case of multiple-stage polymerization, it is preferable to add after the multiple-stage polymerization. Considering fluctuation in water due to heat generation at the time of polymerization and after polymerization, retention due to process delay, opening of the system at the time of addition of the crosslinking agent, addition of water due to the addition of the crosslinking agent, or the like, the post-polymerization crosslinking agent is preferably added in a region of [water content (immediately after polymerization) $\pm$ 3% by mass] from the viewpoint of water content (to be described later).

Subsequently, the polymer particles (for example, polymer particles containing a polymer having a structural unit derived from an ethylenically unsaturated monomer) are obtained by drying in order to remove water from the obtained hydrogel-like polymer. Examples of a drying method include (a) a method of removing water by performing azeotropic distillation by heating from outside in a state where a hydrogel-like polymer is dispersed in a hydrocarbon dispersion medium, and refluxing the hydrocarbon dispersion medium, (b) a method of taking out a hydrogel-like polymer by decantation and drying under reduced pressure, and (c) a method of filtering the hydrogel-like polymer with a filter and drying under reduced pressure. Among these, it is preferable to use the method (a) due to the simplicity in the production process.

It is possible to adjust the particle diameter of water-absorbent resin particles by adjusting a rotation speed of a stirrer during the polymerization reaction, or by adding a flocculant into the system after the polymerization reaction or in the initial stage of drying. By adding a flocculant, it is possible to increase the particle diameter of the obtained water-absorbent resin particles. As the flocculant, an inorganic flocculant can be used. Examples of the inorganic flocculant (for example, powdered inorganic flocculant) include silica, zeolite, bentonite, aluminum oxide, talc, titanium dioxide, kaolin, clay, and hydrotalcite. From the viewpoint of better flocculation effect, the flocculant is preferably at least one selected from the group consisting of silica, aluminum oxide, talc, and kaolin.

In the reverse phase suspension polymerization, a method of adding the flocculant is preferably a method of preliminarily dispersing a flocculant in a hydrocarbon dispersion medium or water of the same type as that used in the polymerization, and then mixing into a hydrocarbon dispersion medium containing a hydrogel-like polymer under stirring.

The addition amount of the flocculant is preferably 0.001 to 1 part by mass, more preferably 0.005 to 0.5 part by mass, and further more preferably 0.01 to 0.2 parts by mass with respect to 100 parts by mass of the ethylenically unsaturated monomer used for the polymerization. In a case where the addition amount of the flocculant is within the above-mentioned range, water-absorbent resin particles having a target particle size distribution can be easily obtained.

In the production of the water-absorbent resin particles, it is preferable to perform surface crosslinking of a surface portion (surface and in the vicinity of surface) of a hydrogel-like polymer using a surface crosslinking agent in a drying step (water removing step) or any subsequent steps. By performing surface crosslinking, water-absorbent characteristics (such as a water retention amount) and the like of the water-absorbent resin particles are easily controlled. The surface crosslinking is preferably performed at the timing when the hydrogel-like polymer has a specific water content. The timing of surface crosslinking is preferably when the water content of the hydrogel-like polymer is 5% to 50% by mass, more preferably when the water content of the hydrogel-like polymer is 10% to 40% by mass, and further more preferably when the water content of the hydrogel-like polymer is 15% to 35% by mass. The water content (mass%) of the hydrogel-like polymer is calculated by the following formula.

$$\text{Water content} = [\text{Ww}/(\text{Ww} + \text{Ws})] \times 100$$

Ww: Water amount of a hydrogel-like polymer obtained by adding water amount used if necessary when mixing a flocculant, a surface crosslinking agent, or the like to an amount obtained by subtracting water amount discharged to the outside of the system in the drying step, from water amount contained in a monomer aqueous solution before polymerization in the entire polymerization step.

Ws: Solid content calculated from the charged amount of materials such as ethylenically unsaturated monomer, crosslinking agent, and initiator that constitute a hydrogel-like polymer.

Examples of the surface crosslinking agent include compounds having two or more reactive functional groups. Examples of the surface crosslinking agent include polyols such as ethylene glycol, propylene glycol, 1,4-butanediol, trimethylolpropane, glycerin, polyoxyethylene glycol, polyoxypropylene glycol, and polyglycerin; polyglycidyl compounds such as (poly)ethylene glycol diglycidyl ether, (poly)glycerin diglycidyl ether, (poly)glycerin triglycidyl ether, trimethylolpropane triglycidyl ether, (poly)propylene glycol polyglycidyl ether, and (poly)glycerol polyglycidyl ether; haloepoxy compounds such as epichlorohydrin, epibromohydrin, and $\alpha$-methyl epichlorohydrin; isocyanate compounds such as 2,4-tolylene diisocyanate and hexamethylene diisocyanate; oxetane compounds such as 3-methyl-3-oxetane methanol, 3-ethyl-3-oxetane methanol, 3-butyl-3-oxetane methanol, 3-methyl-3-oxetane ethanol, 3-ethyl-3-oxetane ethanol, and 3-butyl-3-oxetane ethanol; oxazoline compounds such as 1,2-ethylenebisoxazoline; carbonate compounds such as ethylene carbonate; and hydroxyalkylamide compounds such as bis[N,N-di($\beta$-hydroxyethyl)]adipamide. The surface crosslinking agent may be used alone, or may be used in combination of two or more kinds thereof. The surface crosslinking agent is preferably a polyglycidyl compound, and more preferably at least one selected from the group consisting of (poly)ethylene glycol diglycidyl ether, (poly)glycerin diglycidyl ether, (poly)glycerin triglycidyl ether, (poly)propylene glycol polyglycidyl ether, and polyglycerol polyglycidyl ether.

The use amount of the surface crosslinking agent is preferably 0.01 to 20 mmol, more preferably 0.05 to 10 mmol, further more preferably 0.1 to 5 mmol, particularly preferably 0.15 to 1 mmol, and extremely preferably 0.2 to 0.5 mmol per 1 mol of the ethylenically unsaturated monomer used for polymerization, from the viewpoint of easily obtaining suitable water-absorbent characteristics (such as a water retention amount).

After the surface crosslinking, it is possible to obtain polymer particles which are surface-crosslinked dried products by distilling off water and a hydrocarbon dispersion medium, drying under heating and reduced pressure, or the like with a known method.

The polymerization reaction can be performed using various stirrers having a stirring blade. As the stirring blade, it is possible to use a flat plate blade, a lattice blade, a paddle blade, a propeller blade, an anchor blade, a turbine blade, a Pfaudler blade, a ribbon blade, a full zone blade, a max blend blade, or the like. A flat plate blade has a shaft (stirring shaft) and a flat plate portion (stirring portion) disposed around the shaft. The flat plate portion may have a slit or the like. In a case where the flat plate blade is used as the stirring blade, it is easy to uniformly perform the crosslinking reaction in polymer particles, and it is easy to adjust the deterioration rate in terms of 3-minute values of non-pressurization DW within a suitable range while maintaining water-absorbent characteristics such as a water retention amount.

In addition to the polymer particles, the water-absorbent resin particles of the present embodiment can further contain additional components such as a gel stabilizer, a metal chelating agent (ethylenediaminetetraacetic acid and a salt thereof, diethylenetriamine pentaacetic acid and a salt thereof, and the like, for example, diethylenetriamine pentaacetic acid pentasodium), and a flowability improver (lubricant). Additional components can be disposed inside the polymer particles, on the surface of the polymer particles, or both thereof.

The water-absorbent resin particles may contain a plurality of inorganic particles disposed on the surface of the polymer particles. For example, by mixing the polymer particles and the inorganic particles, it is possible to dispose the inorganic particles on the surface of the polymer particles. The inorganic particles may be silica particles such as amorphous silica.

In a case where the water-absorbent resin particles include inorganic particles disposed on the surface of the polymer particles, the content of the inorganic particles may be within the following range based on the total mass of the polymer particles. The content of the inorganic particles may be 0.05% by mass or more, 0.1% by mass or more, 0.15% by mass or more, or 0.2% by mass or more. The content of the inorganic particles may be 5.0% by mass or less, 3.0% by mass or less, 1.0% by mass or less, or 0.5% by mass or less.

The inorganic particles here usually have a minute size as compared with the size of the polymer particles. For example, the average particle diameter of the inorganic particles may be 0.1 to 50 $\mu$m, 0.5 to 30 $\mu$m, or 1 to 20 $\mu$m. The average particle diameter can be measured by a pore electric resistance method or a laser diffraction/scattering method depending on the characteristics of the particles.

The absorber of the present embodiment contains the water-absorbent resin particles of the present embodiment. The absorber of the present embodiment may contain a fibrous substance, for example, may be a mixture containing water-absorbent resin particles and the fibrous substance. For example, the structure of the absorber may be a structure in which the water-absorbent resin particles and the fibrous substance are uniformly mixed, may be a structure in which the water-absorbent resin particles are sandwiched between the fibrous substances formed in the shape of a sheet or a layer, or may be other structures.

Examples of the fibrous substance include finely pulverized wood pulp; cotton; cotton linter; rayon; cellulosic fibers such as cellulose acetate; synthetic fibers such as polyamide, polyester and polyolefin; and a mixture of these fibers. The average fiber length of the fibrous substance is usually 0.1 to 10 mm, and may be 0.5 to 5 mm. The fibrous substance may be used alone, or may be used in combination of two or more. As the fibrous substance, hydrophilic fibers can be used.

In order to enhance the morphological retention before and during use of the absorber, the fibers may be adhered to each other by adding an adhesive binder to the fibrous substance. Examples of the adhesive binder include thermal bonding synthetic fibers, hot melt adhesives, and adhesive emulsions. The adhesive binder may be used alone, or may be used in combination of two or more.

Examples of the thermal bonding synthetic fiber include a total fusion type binder such as polyethylene, polypropylene, and an ethylene-propylene copolymer; and a non-total fusion type binder made of a side-by-side or core-sheath structure of polypropylene and polyethylene. In the above-mentioned non-total fusion type binder, only the polyethylene portion can be thermal-bonded.

Examples of the hot melt adhesive include a mixture of a base polymer such as ethylene-vinyl acetate copolymer, styrene-isoprene-styrene block copolymer, styrene-butadiene-styrene block copolymer, styrene-ethylene-butylene-styrene block copolymer, styrene-ethylene-propylene-styrene block copolymer, and amorphous polypropylene with a tackifier, a plasticizer, an antioxidant, or the like.

Examples of the adhesive emulsion include a polymerization product of at least one monomer selected from the group consisting of methyl methacrylate, styrene, acrylonitrile, 2-ethylhexyl acrylate, butyl acrylate, butadiene, ethylene, and vinyl acetate.

The absorber of the present embodiment may contain an inorganic powder (for example, amorphous silica), a deodorant, an antibacterial agent, a dye, a pigment, a fragrance, a sticking agent, or the like. In a case where the water-absorbent resin particles contain inorganic particles, the absorber may contain an inorganic powder in addition to the inorganic particles of the water-absorbent resin particles.

The shape of the absorber of the present embodiment may be a sheet shape, for example. The thickness of the absorber (for example, thickness of the sheet shaped absorber) may be 0.1 to 20 mm or 0.3 to 15 mm.

The content of the water-absorbent resin particles in the absorber may be 2% to 100% by mass, 10% to 80% by mass, or 20% to 60% by mass with respect to a total of the water-absorbent resin particles and the fibrous substance, from the viewpoint of easily obtaining sufficient absorption characteristics.

The content of the water-absorbent resin particles in the absorber is preferably 100 to 1000 g, more preferably 150 to 800 g, and further more preferably 200 to 700 g per 1 m$^2$ of the absorber from the viewpoint of easily obtaining sufficient absorption characteristics. The content of the fibrous substance in the absorber is preferably 50 to 800 g, more preferably 100 to 600 g, and further more preferably 150 to 500 g per 1 m$^2$ of the absorber from the viewpoint of easily obtaining sufficient absorption characteristics.

The absorbent article of the present embodiment includes an absorber of the present embodiment. Examples of other constituent members of the absorbent article of the present embodiment include a core wrap that retains an absorber and prevents falloff or flow of a constituent member of the absorber; a liquid permeable sheet disposed on the outermost part at the side where the liquid to be absorbed enters; and a liquid impermeable sheet disposed on the outermost part at the opposite side to the side where the liquid to be absorbed enters. Examples of the absorbent article include diapers (for example, paper diapers), toilet training pants, incontinence pads, sanitary materials (sanitary napkins, tampons, and the like), sweat pads, pet sheets, portal toilet members, and animal excrement treatment materials.

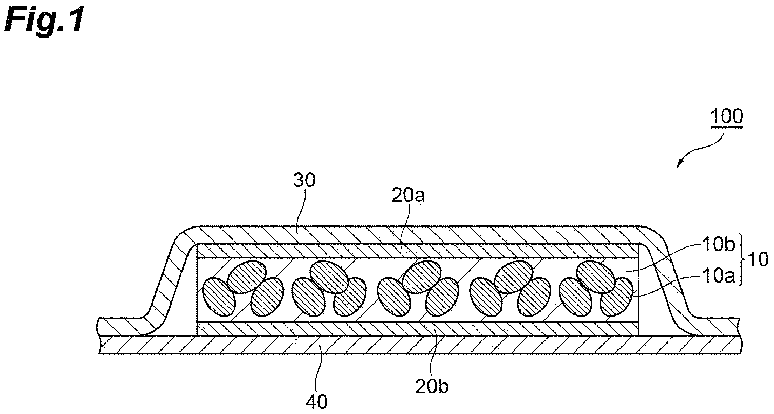

Fig. 1 is a cross-sectional view showing an example of an absorbent article. An absorbent article 100 shown in Fig. 1 includes an absorber 10, core wraps 20a and 20b, a liquid permeable sheet 30, and a liquid impermeable sheet 40. In the absorbent article 100, the liquid impermeable sheet 40, the core wrap 20b, the absorber 10, the core wrap 20a, and the liquid permeable sheet 30 are laminated in this order. In Fig. 1, there is a portion shown so that there is a gap between the members, but the members may be in close contact with each other without the gap.

The absorber 10 has a water-absorbent resin particle 10a of the present embodiment and a fiber layer 10b containing a fibrous substance. The water-absorbent resin particles 10a are dispersed in the fiber layer 10b.

The core wrap 20a is disposed on one surface side of the absorber 10 (an upper side of the absorber 10 in Fig. 1) in a state of being in contact with the absorber 10. The core wrap 20b is disposed on the other surface side of the absorber 10 (a lower side of the absorber 10 in Fig. 1) in a state of being in contact with the absorber 10. The absorber 10 is disposed between the core wrap 20a and the core wrap 20b. Examples of the core wraps 20a and 20b include tissues, non-woven fabrics, woven fabrics, synthetic resin films having liquid permeation holes, and net-like sheets having a mesh. The core wrap 20a and the core wrap 20b each have a main surface having the same size as that of the absorber 10, for example.

The liquid permeable sheet 30 is disposed on the outermost part at the side where the liquid to be absorbed enters. The liquid permeable sheet 30 is disposed on the core wrap 20a in a state of being in contact with the core wrap 20a. Examples of the liquid permeable sheet 30 include a non-woven fabric made of a synthetic resin such as polyethylene, polypropylene, polyester, and polyamide, and a porous sheet. The liquid impermeable sheet 40 is disposed on the outermost part at the opposite side to the liquid permeable sheet 30 in the absorbent article 100. The liquid impermeable sheet 40 is disposed on a lower side of the core wrap 20b in a state of being in contact with the core wrap 20b. Examples of the liquid impermeable sheet 40 include a sheet made of a synthetic resin such as polyethylene, polypropylene, and polyvinyl chloride, and a sheet made of a composite material of these synthetic resins and a non-woven fabric. The liquid permeable sheet 30 and the liquid impermeable sheet 40 have a main surface wider than the main surface of the absorber 10, and outer edges of the liquid permeable sheet 30 and the liquid impermeable sheet 40 are present around the absorber 10 and the core wraps 20a and 20b.

The magnitude relationship between the absorber 10, the core wraps 20a and 20b, the liquid permeable sheet 30, and the liquid impermeable sheet 40 is not particularly limited, and is appropriately adjusted according to the use of the absorbent article or the like. In addition, the method of retaining the shape of the absorber 10 using the core wraps 20a and 20b is not particularly limited, and as shown in Fig. 1, the absorber may be wrapped by a plurality of core wraps, and the absorber is wrapped by one core wrap.

The absorber may be adhered to a top sheet. In a case where the absorber is sandwiched or covered by the core wrap, it is preferable that at least the core wrap and the top sheet are adhered to each other, and it is more preferable that the core wrap and the top sheet are adhered to each other and the core wrap and the absorber are adhered to each other. Examples of a method of adhering the absorber include a method of adhering by applying a hot melt adhesive to the top sheet at predetermined intervals in a striped shape, a spiral shape, or the like in a width direction; and a method of adhering using a water-soluble binder such as starch, carboxymethyl cellulose, polyvinyl alcohol, polyvinylpyrrolidone, and other water-soluble polymers. In addition, in a case where the absorber contains thermal bonding synthetic fibers, a method of adhering by thermal bonding of the thermal bonding synthetic fibers may be adopted.

According to the present embodiment, it is possible to provide a liquid absorbing method using the water-absorbent resin particles, the absorber or the absorbent article of the present embodiment. The liquid absorbing method of the present embodiment includes a step of bringing the liquid to be absorbed into contact with the water-absorbent resin particles, the absorber or the absorbent article of the present embodiment.

According to the present embodiment, it is possible to provide a method for inhibiting liquid leakage in the absorbent article which is a method for inhibiting liquid leakage using the water-absorbent resin particles, the absorber or the absorbent article of the present embodiment. The method for inhibiting liquid leakage of the present embodiment includes an adjustment step of adjusting a deterioration rate in terms of 3-minute values of non-pressurization DW measured by the above-mentioned procedures having (1) to (3) relating to the water-absorbent resin particles of the present embodiment. In the adjustment step, the deterioration rate in terms of 3-minute values of non-pressurization DW can be adjusted to be within each of the above-mentioned ranges (for example, 15% or less).

According to the present embodiment, it is possible to provide a method for producing water-absorbent resin particles, the method including a selection step of selecting water-absorbent resin particles based on a deterioration rate in terms of 3-minute values of non-pressurization DW measured by the above-mentioned procedures having (1) to (3) relating to the water-absorbent resin particles of the present embodiment. In the selection step, the deterioration rate in terms of 3-minute values of non-pressurization DW can be adjusted to be within each of the above-mentioned ranges (for example, 15% or less).

According to the present embodiment, it is possible to provide a method for producing an absorber by using the water-absorbent resin particles obtained by the above-mentioned method for producing water-absorbent resin particles. The method for producing an absorber of the present embodiment includes a particle producing step of obtaining water-absorbent resin particles by the above-mentioned method for producing water-absorbent resin particles. The method for producing an absorber of the present embodiment may include a step of mixing the water-absorbent resin particles and a fibrous substance after the particle producing step. According to the present embodiment, it is possible to provide a method for producing an absorbent article by using the absorber obtained by the above-mentioned method for producing an absorber. The method for producing an absorbent article of the present embodiment includes an absorber producing step of obtaining an absorber by the above-mentioned method for producing an absorber. The method for producing an absorbent article of the present embodiment may include a step of obtaining an absorbent article by using the absorber and other constituent member for an absorbent article after the absorber producing step, and in this step, for example, an absorbent article is obtained by laminating the absorber and other constituent member for an absorbent article with each other.

**Examples**

Hereinafter, the present invention will be more specifically described with reference to examples. However, the present invention is not limited to these examples.

1. Production of water-absorbent resin particles

Example 1

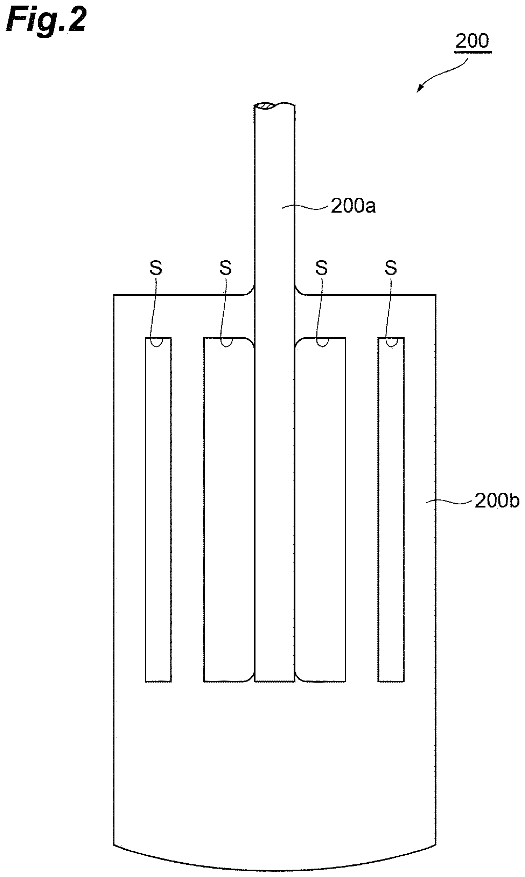

A round-bottomed cylindrical separable flask with the inner diameter of 11 cm and the internal volume of 2 L equipped with a reflux cooling device, a dropping funnel, a nitrogen gas introduction tube, and a stirrer was prepared. The stirrer was equipped with a stirring blade 200 of which an outline is shown in Fig. 2. The stirring blade 200 includes a shaft 200a and a flat plate portion 200b. The flat plate portion 200b is welded to the shaft 200a and has a curved distal end. Four slits S extending along an axial direction of the shaft 200a are formed in the flat plate portion 200b. The four slits S are arranged in a width direction of the flat plate portion 200b, where the width of the two slits S at the inner side is 1 cm, and the width of the two slits S at the outer side is 0.5 cm. The length of the flat plate portion 200b is about 10 cm, and the width of the flat plate portion 200b is about 6 cm. In the prepared separable flask, 293 g of n-heptane, and 0.736 g of a dispersant (maleic anhydride-modified ethylene-propylene copolymer, manufactured by Mitsui Chemicals, Inc., Hi-Wax 1105A) were mixed. The dispersant was dissolved in n-heptane by raising the temperature to 80°C while stirring the mixture in the separable flask with the stirrer. The formed solution was cooled to 50°C.

Meanwhile, 92.0 g (1.03 mol) of an aqueous solution of 80.5% by mass acrylic acid was weighed in a beaker having the internal volume of 300 mL as a water-soluble ethylenically unsaturated monomer, and while cooling from the outside, 147.7 g of an aqueous solution of 20.9% by mass sodium hydroxide was added dropwise to perform 75 mol% of neutralization. Thereafter, 0.092 g of hydroxyethyl cellulose (Sumitomo Seika Chemicals Co., Ltd., HEC AW-15F) as a thickener, 0.0648 g (0.272 mmol) of sodium persulfate as a water-soluble radical polymerization initiator, and 0.0156 g (0.090 mmol) of ethylene glycol diglycidyl ether as an internal crosslinking agent were added in the above-mentioned beaker and dissolved to prepare a first stage aqueous liquid.

Then, the aqueous liquid prepared above was added into the separable flask, and stirring was performed for 10 minutes. Separately, 0.736 g of sucrose stearic acid ester (Mitsubishi-Chemical Foods Corporation, Ryoto Sugar Ester S-370, HLB: 3) as a surfactant was heat-dissolved in 6.62 g of n-heptane to obtain a surfactant solution. The above-mentioned surfactant solution was added into the above-mentioned flask, and the inside of the system was sufficiently replaced with nitrogen while stirring at the rotation speed of 350 rpm of the stirrer. Thereafter, the above-mentioned flask was immersed in a water bath at 70°C to raise the temperature, and polymerization was performed for 60 minutes to obtain a first stage polymerization slurry solution.

Meanwhile, 128.8 g (1.44 mol) of an aqueous solution of 80.5% by mass acrylic acid was weighed in another beaker having the internal volume of 500 mL as a water-soluble ethylenically unsaturated monomer, and while cooling from the outside, 159.0 g of an aqueous solution of 27% by mass sodium hydroxide was added dropwise to perform 75 mol% of neutralization. Thereafter, 0.0907 g (0.381 mmol) of sodium persulfate as a water-soluble radical polymerization initiator and 0.0129 g (0.074 mmol) of ethylene glycol diglycidyl ether as an internal crosslinking agent were added into the above-mentioned beaker and dissolved to prepare a second stage aqueous liquid.

While stirring at the rotation speed of 650 rpm of the stirrer, the inside of the above-mentioned separable flask system was cooled to 25°C, and then the total amount of the above-mentioned second stage aqueous liquid was added to the first stage polymerization slurry solution, and the inside of the system was replaced with nitrogen for 30 minutes. Thereafter, the flask was immersed again in a water bath at 70°C to raise the temperature, and the polymerization reaction was performed for 60 minutes to obtain a hydrogel-like polymer.

To the hydrogel-like polymer after the second stage polymerization, 0.589 g of an aqueous solution of 45% by mass diethylenetriamine pentaacetic acid pentasodium was added under stirring. Thereafter, the flask was immersed in an oil bath set at 125°C, and 254.5 g of water was extracted to the outside of the system while refluxing n-heptane by azeotropic distillation of n-heptane and water. Thereafter, 4.42 g (0.507 mmol) of an aqueous solution of 2% by mass ethylene glycol diglycidyl ether was added into the flask as a surface crosslinking agent, and the mixture was maintained at 83°C for 2 hours.

Thereafter, drying was performed by evaporating n-heptane at 125°C to obtain polymer particles (dried product). These polymer particles were passed through a sieve having the opening of 850 $\mu$m, and 0.5% by mass of amorphous silica (Oriental Silicas Corporation, Tokusil NP-S) with respect to the mass of the polymer particles was mixed to obtain 231.8 g of water-absorbent resin particles containing amorphous silica. The median particle diameter of the water-absorbent resin particles was 368 $\mu$m.

Example 2

231.2 g of water-absorbent resin particles was obtained in the same manner as in Example 1, except that, in preparation of a first stage aqueous liquid, 0.010 g (0.057 mmol) of ethylene glycol diglycidyl ether was added as an internal crosslinking agent; in preparation of a second stage aqueous liquid, 0.0116 g (0.067 mmol) of ethylene glycol diglycidyl ether was added as an internal crosslinking agent; the amount of water extracted by azeotropic distillation of n-heptane and water was 257.2 g; and 0.2% by mass of amorphous silica with respect to the mass of the polymer particles was mixed with the polymer particles. The median particle diameter of the water-absorbent resin particles was 359 $\mu$m.

Example 3

229.0 g of water-absorbent resin particles was obtained in the same manner as in Example 1, except that, in preparation of a first stage aqueous liquid, 0.010 g (0.057 mmol) of ethylene glycol diglycidyl ether was added as an internal crosslinking agent; the rotation speed of the stirrer when obtaining a first stage polymerization slurry solution was changed to 425 rpm; in preparation of a second stage aqueous liquid, 0.0116 g (0.067 mmol) of ethylene glycol diglycidyl ether was added as an internal crosslinking agent; the amount of water extracted by azeotropic distillation of n-heptane and water was 271.0 g; and 0.2% by mass of amorphous silica with respect to the mass of the polymer particles was mixed with the polymer particles. The median particle diameter of the water-absorbent resin particles was 360 $\mu$m.

Example 4

232.0 g of water-absorbent resin particles was obtained in the same manner as in Example 1, except that, in preparation of a first stage aqueous liquid, 0.010 g (0.057 mmol) of ethylene glycol diglycidyl ether was added as an internal crosslinking agent; the rotation speed of the stirrer when obtaining a first stage polymerization slurry solution was changed to 425 rpm; in preparation of a second stage aqueous liquid, 0.0116 g (0.067 mmol) of ethylene glycol diglycidyl ether was added as an internal crosslinking agent; when obtaining a hydrogel-like polymer, after cooling the inside of the separable flask system to 31°C, the total amount of the second stage aqueous liquid was added to the first stage polymerization slurry solution; the amount of water extracted by azeotropic distillation of n-heptane and water was 275.8 g; and 0.2% by mass of amorphous silica with respect to the mass of the polymer particles was mixed with the polymer particles. The median particle diameter of the water-absorbent resin particles was 148 $\mu$m.

Comparative Example 1

232.5 g of water-absorbent resin particles was obtained in the same manner as in Example 1 except that, a stirring blade having two stages of four inclined paddle blades with the blade diameter of 5 cm was used as a stirring blade; in preparation of a first stage aqueous liquid, 0.092 g (0.339 mmol) of 2,2'-azobis(2-amidinopropane) dihydrochloride and 0.018 g (0.068 mmol) of potassium persulfate were added as a water-soluble radical polymerization initiator, and 0.0046 g (0.026 mmol) of ethylene glycol diglycidyl ether was added as an internal crosslinking agent; the rotation speed of the stirrer when obtaining a first stage polymerization slurry was changed to 550 rpm; in preparation of a second stage aqueous liquid, 0.129 g (0.475 mmol) of 2,2'-azobis(2-amidinopropane) dihydrochloride and 0.026 g (0.095 mmol) of potassium persulfate were added as a water-soluble radical polymerization initiator, and 0.0116 g (0.067 mmol) of ethylene glycol diglycidyl ether was added as an internal crosslinking agent; the rotation speed of the stirrer when obtaining a hydrogel-like polymer was changed to 1000 rpm; the amount of water extracted by azeotropic distillation of n-heptane and water was changed to 207.9 g; and 0.2% by mass of amorphous silica with respect to the mass of the polymer particles was mixed with the polymer particles. The median particle diameter of the water-absorbent resin particles was 360 μm.

Comparative Example 2

229.1 g of water-absorbent resin particles was obtained in the same manner as in Example 1 except that, a stirring blade having two stages of four inclined paddle blades with the blade diameter of 5 cm was used as a stirring blade; in preparation of a first stage aqueous liquid, 0.092 g (0.339 mmol) of 2,2'-azobis(2-amidinopropane) dihydrochloride and 0.018 g (0.068 mmol) of potassium persulfate were added as a water-soluble radical polymerization initiator, and 0.0046 g (0.026 mmol) of ethylene glycol diglycidyl ether was added as an internal crosslinking agent; the rotation speed of the stirrer when obtaining a first stage polymerization slurry was changed to 550 rpm; in preparation of a second stage aqueous liquid, 0.129 g (0.475 mmol) of 2,2'-azobis(2-amidinopropane) dihydrochloride and 0.026 g (0.095 mmol) of potassium persulfate were added as a water-soluble radical polymerization initiator, and 0.0116 g (0.067 mmol) of ethylene glycol diglycidyl ether was added as an internal crosslinking agent; the rotation speed of the stirrer when obtaining a hydrogel-like polymer was changed to 1000 rpm; the amount of water extracted by azeotropic distillation of n-heptane and water was changed to 207.9 g; and 0.2% by mass of amorphous silica with respect to the mass of the polymer particles was mixed with the polymer particles. The median particle diameter of the water-absorbent resin particles was 355 μm.

2. Evaluation

Physiological saline retention amount

The physiological saline retention amount (room temperature, 25°C ± 2°C) of the water-absorbent resin particles was measured by the following procedure. First, a cotton bag (cotton broadcloth No. 60, 100 mm in width × 200 mm in length) into which 2.0 g of the water-absorbent resin particles had been weighed was placed in a beaker having the capacity of 500 mL. 500 g of an aqueous solution of 0.9% by mass sodium chloride (physiological saline) was poured in the cotton bag containing the water-absorbent resin particles at one time so that lumps could not be produced, the upper part of the cotton bag was bound with a rubber band, and the cotton bag was left to stand for 30 minutes to swell the water-absorbent resin particles. The cotton bag after an elapse of 30 minutes was dehydrated for 1 minute using a dehydrator (manufactured by KOKUSAN Co., Ltd., product number: H-122) which had been set to have the centrifugal force of 167 G, and the mass Wa (g) of the cotton bag containing the swollen gel after dehydration was measured. The same operation was performed without addition of the water-absorbent resin particles, the empty mass Wb (g) at the time when the cotton bag was wet was measured, and the physiological saline retention amount was calculated from the following formula.

$$\text{Physiological saline retention amount (g/g)} = [Wa - Wb]/2.0$$

Median particle diameter 50 g of the water-absorbent resin particles was used for median particle diameter measurement. The measurement was performed in the environment of the temperature of 25°C ± 2°C and the humidity of 50% ± 10%.

JIS standard sieves were combined in the following order from the top: a sieve having the opening of 850 μm, a sieve having the opening of 500 μm, a sieve having the opening of 425 μm, a sieve having the opening of 300 μm, a sieve having the opening of 250 μm, a sieve having the opening of 180 μm, a sieve having the opening of 150 μm, and a tray.

The water-absorbent resin particles were put in the topmost sieve among the combined sieves, and classification was performed using a Ro-tap shaker (manufactured by Iida-seisakusho Japan Corporation) according to JIS Z 8815 (1994). After the classification, the mass of the water-absorbent resin particles remaining on each of the sieves was calculated as a mass percentage with respect to the total amount to determine a particle size distribution. The relationship between the opening of the sieve and the integrated value of the mass percentage of the water-absorbent resin particles remaining on the sieve was plotted on a logarithmic probability paper by integrating in the order from the one having the largest particle diameter on the sieve with respect to this particle size distribution. By connecting the plots on the probability paper with a straight line, the particle diameter corresponding to the cumulative mass percentage of 50% by mass was defined as the median particle diameter.

Measurement of deterioration rate

The following deterioration treatment was performed in the environment of the temperature of 25°C and the humidity of 50% $\pm$ 10%. 15 g of the water-absorbent resin particles was injected into a mixing container made of stainless steel, having an opening, and having the volume of 1 L, the inlet inner diameter of 4.1 cm, the body inner diameter of 10.0 cm, and the height of 20.6 cm. A lid made of stainless steel was put on the opening of the mixing container so as not to spill the water-absorbent resin particles. The deterioration treatment was performed on the water-absorbent resin particles by setting the mixing container, to which the water-absorbent resin particles were put, on a cross rotary mixer (manufactured by Meiwa Kogyo Co., Ltd., CM-3 type), and pivoting the mixing container in which the water-absorbent resin particles were disposed for 2 hours under conditions of the rotation speed of 45 rpm and the revolution speed of 45 rpm. Fluctuations of $\pm$ 2 rpm were allowed for both rotation speed and revolution speed.

Fig. 4 is a schematic view showing the direction of rotation and the direction of revolution of the mixing container. A direction a in Fig. 4 (clockwise direction when viewed from above the mixing container 300) indicates the direction of rotation, and a direction b in Fig. 4 indicates the direction of revolution. As shown in Fig. 4, the mixing container 300 pivots (rotates) around a rotation axis C1 and pivots (revolves) around a revolution axis C2. The rotation axis C1 is the central axis in a height direction of the mixing container 300. The revolution axis C2 is located at the center in a height direction of the mixing container 300 and is an axis orthogonal to the rotation axis.

The deterioration rate in terms of 3-minute values of non-pressurization DW is a value calculated by the following formula, when I is a 3-minute value of non-pressurization DW of the water-absorbent resin particles before the deterioration treatment (original particles) and II is a 3-minute value of non-pressurization DW of the water-absorbent resin particles after the deterioration treatment.

$$[(I - II)/I] \times 100$$

The deterioration rate in terms of a 10-minute value of non-pressurization DW is a value calculated by the following formula, when III is a 10-minute value of non-pressurization DW of the water-absorbent resin particles before the deterioration treatment (original particles) and IV is a 10-minute value of non-pressurization DW of the water-absorbent resin particles after the deterioration treatment.

$$[(III - IV)/III] \times 100$$

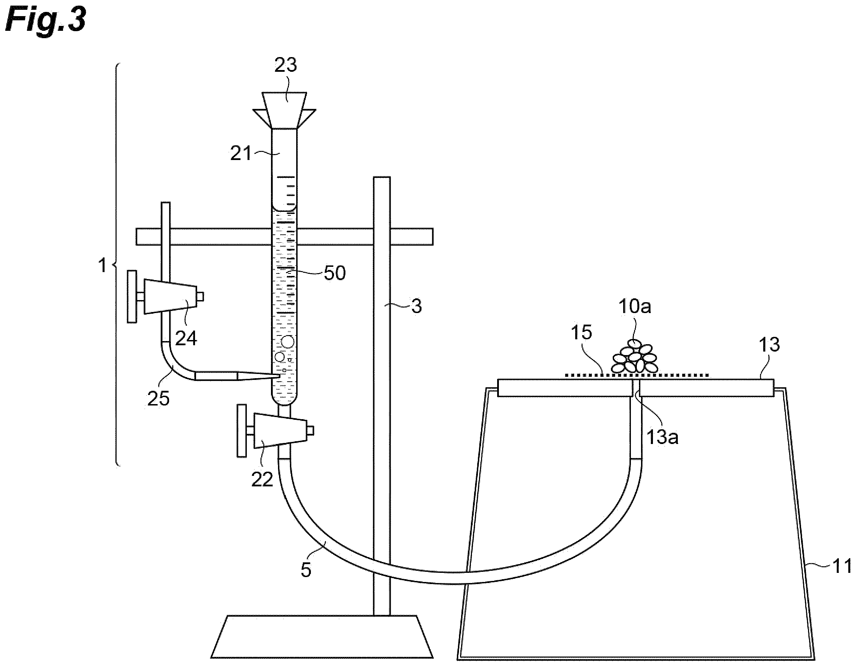

The non-pressurization DW of the particles of water-absorbent resin after the deterioration treatment was measured using a measurement device shown in Fig. 3. The measurement was performed three times for one type of water-absorbent resin particles, and an average value of the measurement values was obtained.

The measurement device has a burette unit 1, a conduit 5, a measurement table 13, a nylon mesh sheet 15, a stand 11, and a clamp 3. The burette unit 1 has a burette tube 21 on which a scale is engraved, a rubber stopper 23 for sealing the opening at the upper part of the burette tube 21, a cock 22 connected to the distal end of the lower part of the burette tube 21, an air introduction tube 25 connected to the lower part of the burette tube 21, and a cock 24. The burette unit 1 is fixed by the clamp 3. The flat plate-shaped measurement table 13 has a through hole 13a having the diameter of 2 mm and formed in the central portion thereof, and is supported by the height-variable stand 11. The through hole 13a of the measurement table 13, and the cock 22 of the burette unit 1 are connected by the conduit 5. The inner diameter of the conduit 5 is 6 mm.

The measurement was performed in the environment of the temperature of 25°C and the humidity of 50% $\pm$ 10%. First, the cock 22 and the cock 24 of the burette unit 1 were closed, and 0.9% by mass saline 50 adjusted to 25°C was put into the burette tube 21 from the opening at the upper part of the burette tube 21. The concentration 0.9% by mass of the saline is a concentration based on the mass of the saline. The opening of the burette tube 21 was sealed with the rubber stopper 23, and then the cock 22 and the cock 24 were opened. The inside of the conduit 5 was filled with the 0.9% by mass saline 50 to prevent air bubbles from entering. The height of the measurement table 13 was adjusted so that the height of the water surface of the 0.9% by mass saline reached the inside of the through hole 13a was the same as the height of the upper surface of the measurement table 13. After the adjustment, the height of the water surface of the 0.9% by mass saline 50 in the burette tube 21 was read by the scale on the burette tube 21, and this position was defined as a zero point (value read at 0 seconds).

The nylon mesh sheet 15 (100 mm × 100 mm, 250 mesh, thickness about 50 μm) was laid in the vicinity of the through hole 13a on the measurement table 13, and a cylinder having the inner diameter of 30 mm and the height of 20 mm was placed on the central portion thereof. 1.00 g of water-absorbent resin particles 10a were uniformly scattered in this cylinder. Thereafter, the cylinder was carefully removed to obtain a sample in which the water-absorbent resin particles 10a were dispersed in a circle shape in the central portion of the nylon mesh sheet 15. Then, the nylon mesh sheet 15 on which the water-absorbent resin particles 10a were placed was moved at a high speed to the extent that the water-absorbent resin particles 10a did not dissipate so that the center of the nylon mesh sheet was at the position of the through hole 13a, and the measurement was started. The timing when air bubbles were first introduced from the air introduction tube 25 into the burette tube 21 was defined as the start of water absorption (0 seconds).

The amount of reduction in the 0.9% by mass saline 50 in the burette tube 21 (that is, the amount of the 0.9% by mass saline absorbed by the water-absorbent resin particles 10a) was sequentially read by units of 0.1 mL, and the amount of reduction Wc (g) of the 0.9% by mass saline 50 was read after 3 minutes and 10 minutes calculating from the start of water absorption by the water-absorbent resin particles 10a. The 3-minute value and 10-minute value of non-pressurization DW were obtained from Wc by the following formula. The non-pressurization DW is a water absorption amount per 1.00 g of the water-absorbent resin particles 10a.

$$\text{Value of non-pressurization DW (mL/g)} = Wc/1.00$$

Evaluation of absorbent article

Evaluation of liquid leakage during use

Artificial urine having the following composition was prepared.

Composition of artificial urine

- Deionized water 5919.6 g
- NaCl 60.0g
- $CaCl_2 \cdot H_2O$ 1.8g
- $MgCl_2 \cdot 6H_2O$ 3.6g
- Food Blue No. 1 (for coloration)
- 1% -Triton X-100 15.0g

The following instruments were prepared.

- Doll manufactured by Kato Tech Co., Ltd.
- Liquid sending pump manufactured by INTEGRA Biosciences AG. (DOSE IT P910, injection slot diameter 0.5 cmφ)
- Tube (inner diameter 0.5 cm)
- Electronic balance
- Bat
- Graduated cylinder
- Constant-temperature tank

The graduated cylinder was used at the time of calibration.

The liquid sending pump manufactured by INTEGRA Biosciences AG. was assembled. A test condition method in which the test solution injection amount was 40 mL and the injection rate was 8 mL/sec was set. Artificial urine was sent to the distal end of the tube. The distal end of the tube was put into the graduated cylinder, and the artificial urine was put into the graduated cylinder by starting the liquid sending pump. The value of the graduated cylinder into which the artificial urine was put was read, and calibration was completed by confirming that the value was the set value (40 mL).

Production of absorbent article 10 g of the water-absorbent resin particles and 8 g of pulverized pulp were uniformly mixed by air papermaking using an air flow type mixer (Padformer manufactured by O-tec Co., Ltd.), and thereby a sheet shaped absorber having the size of 40 cm × 12 cm was produced. Subsequently, in a state where the sheet shaped absorber was sandwiched from its upper and lower sides with two sheets of tissue paper having the basis weight of 16 g/m$^2$ and having the same size as that of the absorber, a laminate was obtained by applying the load of 196 kPa to the entire absorber for 30 seconds to press.

From a diaper (trade name: Pampers Smooth Care, tape type, L size, purchased in 2018)) manufactured by Procter & Gamble Japan K.K., the original top sheet and absorption core were removed, and a back sheet having a pair of side gathers adhered to each of both ends in a lateral direction was collected. The side gathers were rolled up in a lateral direction so that the central portion of the back sheet was exposed. Next, with glue tape, so as not to cover waist gathers, a polyethylene sheet (12 cm × 42 cm) was bonded to the central region, excluding both ends (width: 3 cm) in a longitudinal direction in the back sheet, to reinforce. Next, the absorber produced in the same manner described above was disposed in the center of the polyethylene sheet. Subsequently, after disposing the glue tape on the back sheet along the outer circumferential portion of the absorber, an air-through type porous liquid permeable sheet (15.5 cm × 47 cm) made of polyethylene-polypropylene and having the basis weight of 22 g/m$^2$ was bonded to the back sheet from the upper part of the absorber. In addition, a portion (rolled up portion) of the central portion side of the back sheet in the pair of side gathers was returned to the original position while bonding to the liquid permeable sheet with the glue tape, and an absorbent article (diaper) in a state where the portion overlapped the liquid permeable sheet was obtained.

Measurement of liquid absorption amount of absorbent article

A doll (mannequin for girls, upright, a human body model for children from the lower abdomen to the thigh, transparent) manufactured by Kato Tech Co., Ltd. was prepared in the environment of the temperature of 25°C ± 2°C and the humidity of 50% ± 10%. The dimensions of the doll are as follows. The above-described test solution was adjusted to the temperature of 25°C ± 2°C in a constant-temperature tank. After connecting a tube linked to the urination part of the doll and a tube of the liquid sending pump, the test solution was sent to a liquid outlet of the urination part of the doll.

The length around the abdomen (around the navel position): 475 mm

The length around the hips: 465 mm

The length around the base of the leg: 280 mm

The length between the thighs (length between the bases of both legs): 36 mm

The length between the navel and the urination part: 145 mm

After measuring the mass W1 of the above-mentioned absorbent article, the absorbent article was worn on the doll. For a fixing position of a fastening tape in the absorbent article, a tape stopping position "1" printed on the surface of the back sheet was adopted. At this time, it was confirmed that the gathers of the absorbent article were in contact with the thigh part of the doll, that the gathers were raised up, that there is no bias in the length of the worn part in the front and rear of the absorbent article, and that there is no looseness around the back and the abdomen.

The doll was placed in the bat on its back. 40 mL of the test solution was sent every 5 minutes at the injection rate of 8 mL/sec, and the sending was repeated until leakage from the absorbent article (liquid outflow from the back side or thigh side) occurred. After the leakage occurred, the absorbent article was removed from the doll, and the mass W2 of the absorbent article was measured. Then, the liquid absorption amount "W2 - W1" of the test solution was calculated. The results are shown in Table 1. It is shown that, in a case where the liquid absorption amount is large, the liquid amount that can be absorbed until leakage occurs becomes large.

Table 1

| | Water-absorbent resin particles | | | | | | | Absorbent article |
|---|---|---|---|---|---|---|---|---|
| | Before deterioration treatment (original particles) | | | After deterioration treatment | | Deterioration rate | | Liquid absorption amount [g] |
| | Water retention amount [g/g] | DW 3-min. value [mL/g] | DW 10-min. value [mL/g] | DW 3-min. value [mL/g] | DW 10-min. value [mL/g] | DW 3-min. value [%] | DW 10-min. value [%] | |
| Ex. 1 | 35 | 51 | 57 | 45 | 56 | 12 | 2 | 280 |
| Ex. 2 | 41 | 27 | 60 | 27 | 60 | 0 | 0 | 310 |
| Ex. 3 | 50 | 23 | 65 | 22 | 64 | 4 | 2 | 308 |
| Ex. 4 | 54 | 11 | 37 | 11 | 37 | 0 | 0 | 306 |
| Comp. Ex. 1 | 34 | 27 | 44 | 12 | 43 | 56 | 2 | 230 |
| Comp. Ex. 2 | 44 | 19 | 47 | 11 | 46 | 42 | 2 | 230 |

Before and after the deterioration treatment, the deterioration rate in terms of 3-minute values of non-pressurization DW of the examples was sufficiently smaller than that of the comparative examples. All of the deterioration rates in terms of the 10-minute value of non-pressurization DW before and after the deterioration treatment were about the same levels. From these results, it is presumed that the deterioration rate in terms of 3-minute values of non-pressurization DW is an effective index for inhibiting liquid leakage during use.

**Reference Signs List**

1: burette unit, 3: clamp, 5: conduit, 10: absorber, 10a: water-absorbent resin particle, 10b: fiber layer, 11: stand, 13: measurement table, 13a: through hole, 15: nylon mesh sheet, 20a, 20b:

core wrap, 21: burette tube, 22: cock, 23: rubber stopper, 24: cock, 25: air introduction tube, 50: 0.9% by mass saline, 30: liquid permeable sheet, 40: liquid impermeable sheet, 100: absorbent article, 200: stirring blade, 200a: shaft, 200b: flat plate portion, 300: mixing container, S: slit.

**Claims**

1. Water-absorbent resin particles,
   wherein a deterioration rate in terms of 3-minute values of non-pressurization DW is 15% or less, the deterioration rate being measured by the following procedure:

   (1) 15 g of water-absorbent resin particles is disposed in a mixing container having a volume of 1 L and made of stainless steel;
   (2) deterioration treatment is performed on the water-absorbent resin particles by pivoting the mixing container in which the water-absorbent resin particles are disposed for 2 hours under conditions of a rotation speed of 45 rpm and a revolution speed of 45 rpm; and
   (3) a deterioration rate in terms of 3-minute value of non-pressurization DW calculated by the following formula is obtained, wherein I is a 3-minute value of non-pressurization DW of the water-absorbent resin particles before the deterioration treatment and II is a 3-minute value of non-pressurization DW of the water-absorbent resin particles after the deterioration treatment,

$$[(I - II)/I] \times 100.$$

2. The water-absorbent resin particles according to claim 1, wherein a physiological saline retention amount is more than 40 g/g.

3. An absorber comprising the water-absorbent resin particles according to claim 1 or 2.

4. An absorbent article comprising the absorber according to claim 3.

5. The absorbent article according to claim 4, which is a diaper.

Fig.1

## Fig.2

# Fig.3

EP 3 960 795 A1

# Fig.4

## INTERNATIONAL SEARCH REPORT

| International application No. |
| --- |
| **PCT/JP2020/016751** |

**A.    CLASSIFICATION OF SUBJECT MATTER**

*C08J 3/24*(2006.01)i; *A61F 13/53*(2006.01)i; *C08J 3/12*(2006.01)i
FI:    C08J3/24 Z; A61F13/53 300; C08J3/12 Z

According to International Patent Classification (IPC) or to both national classification and IPC

**B.    FIELDS SEARCHED**

Minimum documentation searched (classification system followed by classification symbols)

C08J3/00-3/28; C08J99/00; A61F13/15-13/84; A61L15/16-15/64

Documentation searched other than minimum documentation to the extent that such documents are included in the fields searched

Published examined utility model applications of Japan 1922-1996
Published unexamined utility model applications of Japan 1971-2020
Registered utility model specifications of Japan 1996-2020
Published registered utility model applications of Japan 1994-2020

Electronic data base consulted during the international search (name of data base and, where practicable, search terms used)

**C.    DOCUMENTS CONSIDERED TO BE RELEVANT**

| Category* | Citation of document, with indication, where appropriate, of the relevant passages | Relevant to claim No. |
| --- | --- | --- |
| X | WO 2009/054197 A1 (SUMITOMO SEIKA CHEMICALS COMPANY, LTD.) 30 April 2009 (2009-04-30) claims, paragraphs [0008], [0012], [0050], [0060]-[0103] | 1-5 |
| X | JP 2007-321007 A (NIPPON SHOKUBAI CO., LTD.) 13 December 2007 (2007-12-13) claims, paragraphs [0013], [0016], [0044]-[0048], [0087], [0093], [0102]-[0149] | 1-5 |
| A | JP 2003-026706 A (SUMITOMO SEIKA CHEMICALS COMPANY, LTD.) 29 January 2003 (2003-01-29) entire text | 1-5 |

☐ Further documents are listed in the continuation of Box C.    ☑ See patent family annex.

| | |
| --- | --- |
| *    Special categories of cited documents:<br>"A"    document defining the general state of the art which is not considered to be of particular relevance<br>"E"    earlier application or patent but published on or after the international filing date<br>"L"    document which may throw doubts on priority claim(s) or which is cited to establish the publication date of another citation or other special reason (as specified)<br>"O"    document referring to an oral disclosure, use, exhibition or other means<br>"P"    document published prior to the international filing date but later than the priority date claimed | "T"    later document published after the international filing date or priority date and not in conflict with the application but cited to understand the principle or theory underlying the invention<br>"X"    document of particular relevance; the claimed invention cannot be considered novel or cannot be considered to involve an inventive step when the document is taken alone<br>"Y"    document of particular relevance; the claimed invention cannot be considered to involve an inventive step when the document is combined with one or more other such documents, such combination being obvious to a person skilled in the art<br>"&"    document member of the same patent family |

| Date of the actual completion of the international search | Date of mailing of the international search report |
| --- | --- |
| **08 July 2020** | **21 July 2020** |

| Name and mailing address of the ISA/JP | Authorized officer |
| --- | --- |
| **Japan Patent Office**<br>**3-4-3, Kasumigaseki, Chiyoda-ku, Tokyo**<br>**100-8915 Japan** | |
| | Telephone No. |

Form PCT/ISA/210 (second sheet) (January 2015)

## INTERNATIONAL SEARCH REPORT
### Information on patent family members

International application No.

**PCT/JP2020/016751**

| Patent document cited in search report | | | Publication date (day/month/year) | Patent family member(s) | Publication date (day/month/year) |
|---|---|---|---|---|---|
| WO | 2009/054197 | A1 | 30 April 2009 | US 2010/0256308 A1 claims, paragraphs [0009], [0012], [0062], [0071]-[0119] EP 2204388 A1 CN 101835814 A KR 10-2010-0087167 A | |
| JP | 2007-321007 | A | 13 December 2007 | (Family: none) | |
| JP | 2003-026706 | A | 29 January 2003 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H06345819 A

- JP H09510889 B